(19) **Europäisches Patentamt**
**European Patent Office**
**Office européen des brevets**

(11) **EP 4 632 076 A1**

(12) **EUROPEAN PATENT APPLICATION**
published in accordance with Art. 153(4) EPC

(43) Date of publication:
**15.10.2025 Bulletin 2025/42**

(21) Application number: **23900673.7**

(22) Date of filing: **06.12.2023**

(51) International Patent Classification (IPC):
**C12Q 1/02** (2006.01)        **C12M 1/00** (2006.01)
**C12M 1/34** (2006.01)

(52) Cooperative Patent Classification (CPC):
**C12M 1/00; C12M 1/34; C12Q 1/02**

(86) International application number:
**PCT/JP2023/043573**

(87) International publication number:
**WO 2024/122559 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.12.2022 JP 2022194670**

(71) Applicant: **KAO CORPORATION**
**Tokyo 103-8210 (JP)**

(72) Inventor: **HORA, Toshiki**
**Wakayama-shi, Wakayama 640-8580 (JP)**

(74) Representative: **Vossius & Partner**
**Patentanwälte Rechtsanwälte mbB**
**Siebertstrasse 3**
**81675 München (DE)**

(54) **CULTIVATION BEHAVIOR PREDICTION METHOD**

(57)     In a culture behavior prediction method, one or more processors capable of using a learned model executes a first step of acquiring a change rate of a specific component as a culture behavior speed index by inputting culture information including the concentration of the specific component in a culture vessel and specific culture condition at a certain culture time point to the learned model, a second step of calculating the concentration of the specific component at a next culture time point using at least the acquired change rate of the specific component, a third step of acquiring the specific culture condition at the next culture time point, and a fourth step of acquiring culture result information over a predetermined culture time by repeating a prediction cycle including the first step, the second step, and the third step related to each of the culture time points.

FIG.3

EP 4 632 076 A1

**Description**

TECHNICAL FIELD

The present invention relates to a technique for predicting a culture behavior in a culture vessel in which a microorganism is cultured.

BACKGROUND ART

Patent Document 1 below discloses a cell culture process search method capable of searching for optimum culture conditions without repeating experiments in cell culture and bioproduction.
This method includes a process condition generating step of generating a plurality of process conditions, a culture result predicting step of acquiring cell culture prediction results for the plurality of process conditions, and an optimization process condition acquisition step of finding an optimum process condition from the culture prediction results. The culture result predicting step includes a process condition acquisition step, an uptake constraint condition acquisition step, an optimization calculation step, and a concentration change calculation step, and the optimization calculation step calculates a metabolic flow rate (consumption rate) with a mathematical model (metabolic circuit model) related to cell metabolism on the basis of a culture medium composition (culture medium component concentration) and an uptake constraint condition.

CITATION LIST

Patent Document 1: WO 2021/166824 A

SUMMARY OF THE INVENTION

According to the present invention, there is provided a culture behavior prediction method for predicting a culture behavior in a culture vessel in which a microorganism are cultured, in which one or more processors capable of using a learned model that outputs a culture behavior speed index using culture information as an input executes: a first step of acquiring a change rate of a specific component as the culture behavior speed index by inputting the culture information including a concentration of the specific component and a specific culture condition in the culture vessel at a certain culture time point to the learned model; a second step of calculating the concentration of the specific component in the culture vessel at a next culture time point using at least the acquired change rate of the specific component; a third step of acquiring the specific culture condition at the next culture time point; and a fourth step of acquiring culture result information over a predetermined culture time by repeating a prediction cycle including the first step, the second step, and the third step related to each of the culture time points.
In addition, according to the present invention, it is possible to provide a culture behavior prediction apparatus including at least a memory and the one or more processors described above, the culture behavior prediction apparatus capable of executing the culture behavior prediction method described above.
In addition, it is also possible to provide a program for causing a computer to execute the culture behavior prediction method described above, and it is also possible to provide a recording medium readable by a computer recording such a program. The recording medium includes a non-transitory tangible medium.

BRIEF DESCRIPTION OF THE DRAWINGS

[Fig. 1] is a schematic diagram illustrating an example of a culture system.
[Fig. 2] is a diagram conceptually illustrating a hardware configuration example of an information processing apparatus capable of executing a culture behavior prediction method according to the present embodiment.
[Fig. 3] is a flow chart for explaining the culture behavior prediction method according to the present embodiment.
[Fig. 4] is a diagram conceptually illustrating a flow of various types of information (data) in a first prediction method.
[Fig. 5] is a diagram conceptually illustrating a flow of various types of information (data) in a second prediction method.
[Fig. 6] is a diagram conceptually illustrating a flow of various types of information (data) in a third prediction method.
[Fig. 7] is a table indicative of prediction accuracy of the first prediction method, the second prediction method, and the third prediction method.

DESCRIPTION OF EMBODIMENTS

The prediction method used in the culture result predicting step in the above-described method described as the background art has room for improvement in prediction accuracy.

The present invention provides a technique capable of predicting a culture behavior in a culture vessel in which a microorganism is cultured.

According to the present invention, it is possible to provide a technique capable of predicting a culture behavior in a culture vessel in which a microorganism is cultured.

Hereinafter, examples of preferred embodiments of the present invention (hereinafter, referred to as the present embodiment) will be described. Note that the present embodiment described below is an example, and the present invention is not limited to the following configuration.

The present embodiment described below is a culture behavior prediction method (hereinafter, referred to as the present method) for predicting a culture behavior in a culture vessel in which a microorganism is cultured.

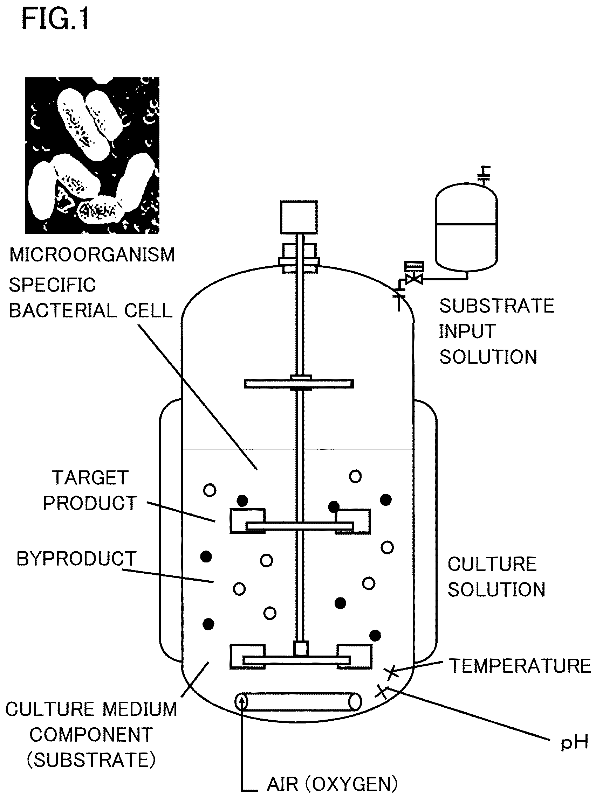

Fig. 1 is a schematic diagram illustrating an example of a culture system.

Fig. 1 exemplifies an example of a culture tank in which liquid culture is performed, and a microorganism is cultured in a culture solution in the culture tank (culture vessel).

In such a culture system, a temperature in the culture tank or the culture solution, a hydrogen ion index (pH) of the culture solution, and the like are measured, and charge of a substrate, which is a nutrient source necessary for the culture of microorganisms, supply of oxygen, and the like are controlled, so that production of a target object (target product) associated with the culture behavior is promoted.

The substrate is charged into the culture tank as a solution, and the solution is expressed as a substrate input solution in the present specification.

Oxygen is supplied by constantly flowing air from an air supply pipe at an arbitrary set flow rate where the air supply pipe is provided in a lower part and an exhaust pipe in an upper part of the culture tank. Furthermore, an oxygen supply efficiency can be increased by stirring at an arbitrary set speed using a stirring blade.

In addition, in the culture behavior, as exemplified in Fig. 1, in addition to the target object, other substances (byproducts, carbon dioxide, and the like) are also generated.

However, the present method is not intended only for the liquid culture exemplified in Fig. 1, but may be intended for solid culture.

In addition, it is sufficient that a type of microorganism and a culture medium can be determined according to a target object to be produced, and the present method does not limit the microorganism to be cultured, the substrate, the target object, and the like.

Microorganisms include, for example, bacterial strains, strains belonging to fungal strains, undifferentiated cells and tissue cultures of animals or plants.

The culture medium can contain, for example, various components generally contained in the culture medium of microorganisms such as a carbon source, a nitrogen source, a metal salt such as a magnesium salt or a zinc salt, a sulfate, a phosphate, a pH adjusting agent, a surfactant, and an antifoaming agent.

Substrates include, for example, saccharides of glucose, sucrose, and fructose, alcohols such as ethanol and methanol, organic acids such as citric acid, malic acid, and succinic acid, and waste molasses, and the like.

Target objects or byproducts include, for example, organic acids, amino acids, alcohols, antibodies, enzymes, coenzymes, vitamins, carbohydrates, fatty acids, and nucleic acids as substances generated by the metabolism of microorganisms.

[Hardware Configuration Example]

The present method is executed by one or more processors included in one or more information processing apparatuses.

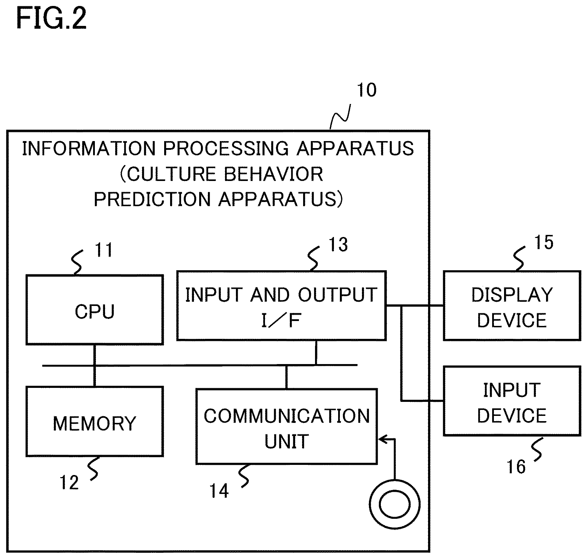

Fig. 2 is a diagram conceptually illustrating a hardware configuration example of an information processing apparatus 10 capable of executing the present method.

The information processing apparatus 10 is a so-called computer, and includes a CPU 11, a memory 12, an input/output interface (I/F) 13, a communication unit 14, and the like. The information processing apparatus 10 may be a stationary personal computer (PC) or a portable terminal such as a portable PC, a smartphone, a tablet, and the like.

The CPU 11 is a so-called processor, and may include an application specific integrated circuit (ASIC), a digital signal processor (DSP), a graphics processing unit (GPU), and the like, in addition to a general central processing unit (CPU). The memory 12 is a random access memory (RAM), a read only memory (ROM), or an auxiliary storage device (such as a hard disk).

The input/output I/F13 can be connected to a user interface device such as a display device 15 and an input device 16. The display device 15 is a device that displays a screen corresponding to drawing data prepared by the CPU 11 or the like, such as a liquid crystal display (LCD) and a cathode ray tube (CRT). The input device 16 is a device that receives an input of a user operation such as a keyboard and a mouse. The display device 15 and the input device 16 may be integrated and realized as a touch panel.

The communication unit 14 performs communication with other computers via a communication network, exchange of signals with other devices such as a printer, and the like. A portable recording medium or the like can also be connected to the communication unit 14.

The hardware configuration of the information processing apparatus 10 is not limited to the example of Fig. 2. The information processing apparatus 10 may include other hardware elements not shown in the drawings. In addition, the number of hardware elements is not limited to the example of Fig. 2. For example, the information processing apparatus 10 may include a plurality of the CPUs 11. In addition, the information processing apparatus 10 may be realized by a plurality units of computers including a plurality of housings.

The information processing apparatus 10 can execute the present method by the CPU 11 executing a computer program stored in the memory 12. In addition, it can also be expressed that the CPU 11 can execute the present method through execution of the computer program stored in the memory 12. This computer program is installed from, for example, a portable recording medium such as a compact disc (CD) and a memory card, or another computer on a network via the input/output I/F13 or the communication unit 14, and then stored in the memory 12.

The information processing apparatus 10 (CPU 11) can use a learned model that outputs a culture behavior speed index using culture information as an input. The learned model can be obtained by machine learning in which teacher data including a plurality of combinations of the culture information and the culture behavior speed index is used. Therefore, the information processing apparatus 10 can be expressed as a culture behavior prediction apparatus capable of executing the culture behavior prediction method, the apparatus being an apparatus capable of using the learned model.

The "culture information" is information related to the culture behavior of the microorganism and is information input to the learned model.

The "culture information" at least includes the concentration of the specific component in the culture vessel and the specific culture condition.

The concentration of the specific component in the culture vessel includes at least a substrate concentration or a target object concentration in the culture solution, and may include a byproduct concentration in the culture solution in addition to one or both of them.

The specific culture condition is the condition that can affect the culture behavior of microorganisms, and includes, for example, the temperature in the culture vessel, the hydrogen ion index (pH) of the culture solution in the culture vessel, an oxygen transfer rate, an oxygen consumption rate, and the like.

The "culture behavior speed index" is an index value indicating a speed in the culture behavior, and can be denoted as a change rate of a specific component in the culture vessel. The change rate of the specific component as the culture behavior speed index means a change rate of a substance amount of a characteristic component per unit bacterial cell mass (wet bacterial cell weight).

In addition, the "specific component" in the culture behavior speed index and the "specific component" of the concentration information included in the culture information are common in all (all components) or some (some components). Therefore, as the culture behavior speed index, the generation rate of the target object or the consumption rate of the substrate is acquired from at least the learned model. In addition, as the culture behavior speed index, a generation rate of a byproduct, a generation rate of carbon dioxide, and the like may be further acquired in addition to one or both of them.

As described above, the learned model is constructed so as to output only the culture behavior speed index using only the culture information as an input, and the culture information and the culture behavior speed index are configured by only an intensive variable or a dimensionless number, or both of them.

Specifically, the concentration of the specific component in the culture vessel included in the culture information is indicated by, for example, a component content weight (g/L) per unit volume of the culture solution, and can be said to be an intensive variable. In addition, the temperature, the hydrogen ion index, the oxygen transfer rate ($mol/m^3/h$), and the oxygen consumption rate ($mol/m^3/h$) as the specific culture condition are also intensive variables or dimensionless numbers. Furthermore, the change rate of the specific component as the culture behavior speed index is indicated, for example, by a change rate of a substance amount (mol/g/h) per unit bacterial cell amount (per 1 g of bacterial cell), and can be said to be an intensive variable.

As described above, by setting input/output of an AI model to an intensive variable or a dimensionless number, culture behavior prediction is possible without depending on a shape or capacity of the culture tank. In other words, even a culture behavior in a culture tank having a different shape and capacity from the culture tank from which teacher data is obtained can be predicted with high accuracy.

The learned model used in the present embodiment may be a regression equation obtained by regression analysis or a neural network model obtained by deep learning or the like, and a data structure, a learning algorithm, and the like of the model are not limited.

For example, the learned model is realized by a combination of a computer program and parameters, a combination of a plurality of functions and parameters, or the like. In a case where the learned model is constructed by a neural network and an input layer, an intermediate layer, and an output layer are regarded as a unit of one neural network, the learned model may refer to one neural network or may refer to a combination of a plurality of neural networks. In addition, the learned model may be configured by a combination of a plurality of multiple regression equations, or may be configured by one multiple regression equation. In a case where the learned model is configured by a combination of a plurality of function models each of which approximately expresses the bacterial cell activity response, each of the function models may be constructed using regression of supervised learning, and each of the function models may be constructed such that at least a part of culture information as an input of the learned model is used as an input variable, and a part of a plurality of types of culture behavior speed indexes as an output of the learned model is used as an output variable.

The learned model may be stored in the memory 12 in the information processing apparatus 10, or may be stored in a memory of another computer accessible by the information processing apparatus 10 through communication.

Note that a concrete learning algorithm for constructing the learned model is not limited at all.

Hereinafter, the learned model used in the present method is denoted as an AI model. In addition, in the following description, an execution subject of the present method is described as the CPU 11.

[Culture Behavior Prediction Method]

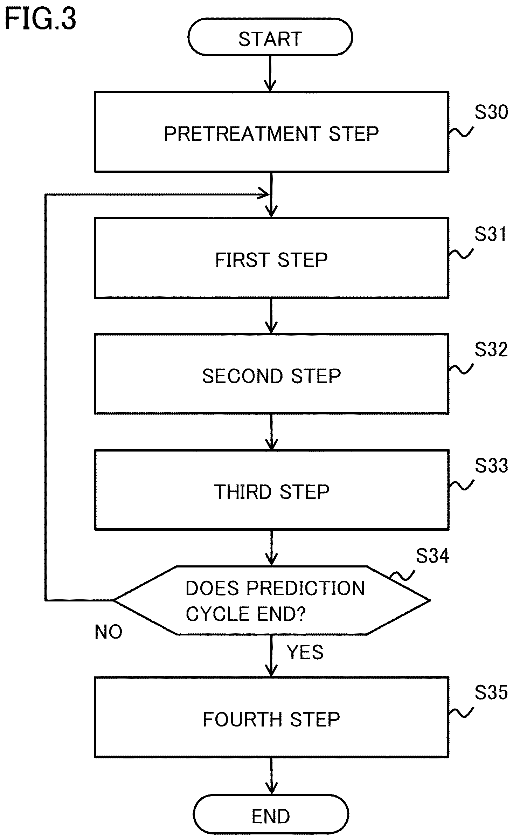

Hereinafter, details of the present method will be described with reference to Fig. 3. Fig. 3 is a flow chart for explaining the culture behavior prediction method according to the present embodiment.

The present method mainly includes repeatedly executing a prediction cycle for each culture time point including a first step (S31), a second step (S32), and a third step (S33), and then executing a fourth step (S35). However, Fig. 3 illustrates a pretreatment step (S30), and an end determination step (S34) of the prediction cycle in order to describe the present method in more detail.

Hereinafter, each of the steps will be described in detail.

In the pretreatment step (S30), the CPU 11 executes pretreatment for executing the prediction cycle. For example, the CPU 11 acquires culture information at a prediction start time point of the culture behavior (initial culture time point (0)) and an initial value (initial culture condition or the like) used in calculation of the second step (S32).

Various types of information acquired in the pretreatment step (S30) are stored in the memory 12 in advance, or stored in a memory of another computer.

In the first step (S31), the CPU 11 acquires the change rate of the specific component as a culture behavior speed index by inputting culture information at a certain culture time point to the AI model.

In a case where the AI model is stored in the memory 12 in the information processing apparatus 10, the CPU 11 can acquire the change rate of the specific component from the AI model by inputting the culture information to the AI model. On the other hand, in a case where the AI model is stored in the memory of another computer accessible by the information processing apparatus 10 through communication, the CPU 11 can acquire the change rate output from the AI model from the computer through communication by sending the culture information to the computer.

The culture information which is the input information of the AI model and the culture behavior speed index which is the output information of the AI model are determined according to the mode of the AI model, and the following examples are given.

First, the culture information at least includes, as described above, the concentration of a specific component in a culture vessel and a specific culture condition. For example, in a case where the culture information includes the concentration of the target object in the culture solution, the AI model is constructed so that at least the generation rate of the target object is acquired as the culture behavior speed index. In addition, in a case where the culture information includes a substrate concentration in the culture solution, the AI model is constructed so that at least the consumption rate of the substrate is acquired as the culture behavior speed index. In addition, in a case where the culture information includes the target object concentration and the substrate concentration in the culture solution, the AI model is constructed so that at least one or both of the generation rate of the target object and the consumption rate of the substrate are acquired as the culture behavior speed index.

In the present embodiment, the culture behavior can be estimated with high accuracy by using such an AI model.

In the present embodiment, the specific culture condition includes the temperature in the culture vessel and the hydrogen ion index (pH) of the culture solution in the culture vessel, and an oxygen transfer rate or an oxygen consumption rate.

The oxygen consumption rate (OCR) means an oxygen consumption rate ($mol/m^3/h$) per unit volume of the culture solution by the microorganism in the culture vessel.

The oxygen transfer rate (OTR) means an oxygen transfer rate ($mol/m^3/h$) per unit volume of the culture solution at which oxygen supplied into the culture vessel moves into the culture solution to become dissolved oxygen.

Regarding the oxygen consumption rate and the oxygen transfer rate, it is sufficient that either one is used in the same manner in the present method. This is because oxygen supply becomes a bottleneck during culture since the rate at which oxygen is dissolved in an aqueous solution (culture solution) is slow. For example, by measuring the oxygen concentration and the air flow rate on each of the supply side and the discharge side of air in the culture system, it is possible to derive, from the difference in the amount of passing oxygen (mol/h) per unit time at each point, how much oxygen has been supplied per unit culture solution amount ($m^3$), that is, the oxygen transfer rate OTR ($mol/m^3/h$). On the other hand, since the amount of oxygen that can be dissolved in the culture solution is very small compared to the oxygen transfer (consumption) rate per unit time, according to the following balance equation of oxygen in the culture solution, the oxygen transfer rate OTR and the oxygen consumption rate OCR can be treated equally by regarding the value related to the dissolved oxygen concentration as almost 0.

$$OTR \ (mol/m^3/h) = OCR \ (mol/m^3/h) + (\text{dissolved oxygen concentration } (g/m^3)/\text{oxygen substance amount } (g/mol))/\Delta t(h)$$

Note that although gaseous oxygen is also present in the culture solution, only the oxygen in the solution is available in the bacterial cells, and the rate at which the oxygen dissolves in the aqueous solution (culture solution) is low. Therefore, the gaseous oxygen in the solution is ignored in the above balance equation.

In addition, the temperature in the culture vessel may be the temperature of the culture solution in the culture vessel, the temperature of the air in the culture vessel, or both of them.

By including such specific culture condition (in particular, the oxygen transfer rate or the oxygen consumption rate) in addition to the concentration of the specific component in the culture vessel in the culture information input to the AI model in this manner, the estimation accuracy of the AI model can be improved, and eventually, the prediction accuracy of the culture behavior can be improved.

However, the specific culture condition may be any one or more of temperature, pH, oxygen transfer rate, and oxygen consumption rate, information other than these, or a combination of them.

The AI model may be constructed such that the culture information further includes bacterial cell activity information indicating the bacterial cell activity state of the microorganism by a categorical variable.

In this case, in the first step (S31), the CPU 11 acquires the change rate of the specific component by inputting culture information including the bacterial cell activity information in addition to the above-described concentration of the specific component and the specific culture condition at a certain culture time point to the AI model.

The bacterial cell activity information indicates, for example, as a bacterial cell activity state of the microorganism in the culture vessel, whether the state is in a growth phase of bacterial cell or in a target object production phase with a categorical variable (0 or 1).

By adding the bacterial cell activity information to the culture information input to the AI model in this manner, the bacterial cell activity response can be simulated in the AI model, and eventually the prediction accuracy of the culture behavior can be further improved.

In a case where the culture information includes bacterial cell activity information, the specific culture condition included in the culture information preferably includes an oxygen transfer rate or an oxygen consumption rate.

This is because the estimation accuracy of the change rate of the specific component in the culture solution can be enhanced by using the oxygen transfer rate or the oxygen consumption rate as the input information of the AI model. Furthermore, since the bacterial cell activity state can be estimated using the oxygen transfer rate or the oxygen consumption rate, the oxygen transfer rate or the oxygen consumption rate can be used for calculating bacterial cell activity information at the next culture time point.

The AI model may be constructed such that the culture information further includes byproduct state information indicating an increase and decrease state of the byproduct in the culture vessel by a categorical variable. In this case, in the first step (S31), the change rate of the specific component and the generation rate of the byproduct are acquired by inputting culture information including the byproduct state information in addition to the above-described concentration of the specific component and the specific culture condition at a certain culture time point to the AI model.

The byproduct state information is information indicating an increase and decrease state of the byproduct accompanying the generation of the byproduct by the microorganism, and is, for example, information indicating whether the state is an increase state or a decrease state with a categorical variable (0 or 1). In this case, the byproduct is a substance corresponding to an intermediate in a metabolic pathway in which bacterial cells produce a target object. Therefore, the increased state of byproducts indicates a metabolic state in which the metabolism after the intermediate is stagnant in the metabolic pathway, and the decreased state of byproducts indicates a metabolic state in which the metabolism after the intermediate is promoted. In addition, the generation rate of the byproduct indicates a positive value in a metabolic state in which the metabolism after the intermediate is stagnant, and indicates a negative value in a metabolic state in which the metabolism after the intermediate is promoted. Note that the specific component in this case indicates a target object or a substrate.

By adding the byproduct state information to the culture information input to the AI model in this manner, the bacterial cell activity response can be simulated in the AI model, and eventually the prediction accuracy of the culture behavior can be further improved.

The second step (S32) and the third step (S33) are preparation steps for the next prediction cycle, and culture information at the next culture time point is acquired.

Specifically, in the second step (S32), the CPU 11 calculates a concentration of the specific component in the culture vessel at the next culture time point using at least the change rate of the specific component acquired in the first step (S31). For example, in a case where the generation rate of a target object is acquired from the AI model in the first step (S31), the CPU 11 calculates the concentration of the target object in the culture solution at the next culture time point using at least the generation rate of the target object.

In the second step (S32), a substance balance equation can be used.

In the example of the following balance equation (Equation 1), $n_{TGT}$ represents the substance amount (mol) of the target object, $r_{TGT}$ represents the generation rate (mol/g/h) of the target object, and B(t) represents the bacterial cell mass (g) of the specific bacterial cell of the microorganism at the culture time point t. In addition, in the example of (Equation 2), $MW_{TGT}$ represents a molecular weight (g/mol) of the target object, V represents the culture solution amount (L) at the culture time point (t+1), and $C_{TGT}$ represents the target object concentration (g/L) in the culture solution at the culture time point (t+1).

In this case, the CPU 11 can calculate the substance amount $n_{TGT}$ of the target object at the next culture time point (t+1) by solving the differential equation of (Equation 1), and can calculate the target object concentration $C_{TGT}$ in the culture solution at the next culture time point (t+1) by substituting the substance amount into (Equation 2).

[Mathematical Equation 1]

$$\frac{dn_{TGT}}{dt} = r_{TGT} \cdot B(t) \qquad \cdots (Equation\ 1)$$

$$C_{TGT} = \frac{n_{TGT} \cdot MW_{TGT}}{V} \qquad \cdots (Equation\ 2)$$

However, the substance balance equation used in the second step (S32) is not limited to such an example.

B(t) and V in the above-describe (Equation 1) and (Equation 2) may be extracted from a value list determined in advance for each culture time point, or may be calculated by a substance balance equation or the like.

For example, as exemplified in the following (Equation 3), the CPU 11 can calculate the specific bacterial cell mass of microorganism at each culture time point on the basis of the culture elapsed time and the oxygen transfer rate or the oxygen consumption rate at each culture time point.

The specific bacterial cell mass calculated here means a wet weight or a dry weight of a specific type of bacterial cell of a microorganism cultured in a culture vessel, and may be mass of one type of bacterial cell or two or more types of bacterial cells.

In (Equation 3), min{} is a function that takes a smaller value between a value on the left side and a value on the right side separated by a comma. The left side shows a state in which the dissolved oxygen in the culture solution is excessive and the specific bacterial cells grow according to a time t, and the right side shows an upper limit state in which the dissolved oxygen is insufficient, the upper limit state being in accordance with the oxygen transfer rate or the oxygen consumption rate.

In addition, in (Equation 3), BCI represents an initial bacterial cell concentration of a specific bacterial cell of a microorganism in the culture vessel, and k1, k2, and k3 each are a fixed value. A value of the BCI is acquired as an initial value in the first step (S31), for example. k1, k2, and k3 are values obtained by experiments by the present inventors, and are, for example, k1 = 0.18, k2 = 17.5, and k3 = 0.334. However, k1, k2, and k3 may be appropriately changed according to the value of BCI, the type of microorganism, or the culture medium.

In (Equation 3), the oxygen transfer rate OTR may be replaced with the oxygen consumption rate OCR.

[Mathematical Equation 2]

$$\frac{B(t)}{V} = min\{BCI \cdot exp(k1 \cdot t),\ k2 \cdot OTR^{k3}\}$$

$$\cdots (\text{Equation 3})$$

In the example of (Equation 3) described above, a culture solution amount V at the next culture time point (t+1) may be extracted from a value list determined in advance or may be calculated by a substance balance equation or the like.

The CPU 11 can calculate the concentration of the specific component at the next culture time point by using at least the specific bacterial cell mass thus calculated and the change rate of the specific component acquired in the first step (S31). When (Equation 1), (Equation 2), and (Equation 3) described above are used, the bacterial cell mass $B(t)$ of the specific bacterial cell of the microorganism at the culture time point t is calculated by (Equation 3), the substance amount $n_{TGT}$ of the target object at the next culture time point (t+1) can be calculated by solving the differential equation of (Equation 1) using the calculated $B(t)$ and generation rate $r_{TGT}$ of the target object acquired in step (S31), and the target object concentration at the next culture time point (t+1) can be calculated using (Equation 2).

In a case where the culture information input to the AI model includes bacterial cell activity information, in the second step (S32), the CPU 11 acquires bacterial cell activity information at the next culture time point.

The next bacterial cell activity information may be acquired from a value list determined in advance for each culture time point, or may be calculated using an oxygen transfer rate or an oxygen consumption rate included in the specific culture condition.

In the latter case, for example, the bacterial cell activity information is set to a value (0) indicating that the bacterial cell activity state is in the growth phase of bacterial cell when the left side is selected (decreases) in the min{} function of (Equation 3) described above, and is set to a value (1) indicating the production phase of the target object when the right side is selected (decreases).

In addition, in a case where the culture information input to the AI model includes the byproduct state information, and the generation rate of the byproduct is acquired from the AI model as one of the change rates of the specific component, in the second step (S32), the CPU 11 can acquire byproduct state information at the next culture time point using the acquired generation rate of the byproduct.

For example, the byproduct state information is set to a value (0) indicating the increasing state of the byproduct in a state where the generation rate of the byproduct is 0 or more, and is set to a value (1) indicating the decreasing state of the byproduct in a case where the generation rate of the byproduct is less than 0.

In the third step (S33), the CPU 11 acquires the specific culture condition at the next culture time point. For example, a list of specific culture condition for each culture time point is stored in advance in the memory 12, and the CPU 11 acquires the specific culture condition at the next culture time point from the list.

In a case where the specific culture condition includes the temperature in the culture vessel, the hydrogen ion index of the culture solution in the culture vessel, and the oxygen transfer rate or the oxygen consumption rate, in the third step (S33), the CPU 11 acquires the specific culture condition including the temperature, the hydrogen ion index, and the oxygen transfer rate or the oxygen consumption rate at the next culture time point.

In the third step (S33), not only the specific culture condition included in the culture information that is the input information of the AI model but also values used for various calculation expressions (including the substance balance equation) used in the calculation of the second step (S32) may be acquired.

In the step (S34), the CPU 11 determines an end condition of the prediction cycle for each culture time point, the prediction cycle including the first step (S31), the second step (S32), and the third step (S33). When the end condition is satisfied (S34; YES), the CPU 11 proceeds to the fourth step (S35), and when the end condition is not satisfied (S34; NO), the first step (S31) and subsequent steps are executed as a prediction cycle of the next culture time point.

As one of the end conditions of the prediction cycle, there is a condition as to whether the execution of the prediction cycle for a predetermined culture time determined in advance has been completed. This can be determined on the basis of whether the number of executions of the prediction cycle has reached a predetermined number of times.

In addition, a condition as to whether or not the substrate concentration in the culture solution in the culture vessel has become a predetermined value (for example, 0) or less may be added to one of the end conditions. In this case, in a case where any one of these two end conditions is satisfied, the process may proceed to the fourth step (S35).

By setting the substrate concentration as the end condition in this manner, the prediction cycle can be stopped as the culture behavior is stopped, so that the prediction accuracy of the culture behavior can be improved.

In addition, a condition as to whether or not the target object concentration in the culture solution in the culture vessel has become a target value may be further added to one of the end conditions. In this case, in a case where any one of these three end conditions is satisfied, the process may proceed to the fourth step (S35). As described above, the present embodiment does not limit the end condition.

In a mode in which the substrate concentration is used for the end condition, the AI model is constructed such that at least the target object concentration is included in the culture information as the input information, and at least the generation rate of the target object and the substrate consumption rate are output as the culture behavior speed index.

In this case, in the first step (S31), the CPU 11 acquires the generation rate of the target object and the substrate consumption rate as the culture behavior speed index by inputting culture information including the target object concentration to the AI model, and in the second step (S32), acquires substrate input information at the culture time point, and further calculates a substrate concentration in the culture vessel at the next culture time point using at least the substrate input information and the substrate consumption rate acquired from the AI model.

A substance balance equation or the like is used for calculating the substrate concentration in the second step (S32).

In the example of the following balance equation (Equation 4), $n_{SBS}$ represents the substance amount (mol) of the substrate, $r_{SBS}$ represents the consumption rate (mol/g/h) of the substrate, and B(t) represents the bacterial cell mass (g) of the specific bacterial cell of the microorganism at the culture time point t, $C_{Feed}$ represents the substrate concentration (g/L) in the substrate input solution, $F_{SBS}(t)$ represents the substrate input speed (L/h) at the culture time point t, and $MW_{SBS}$ represents the molecular weight (g/mol) of the substrate. In addition, in the example of (Equation 5), V represents the culture solution amount (L) at the culture time point (t+1).

In this case, the CPU 11 can acquire $C_{Feed}$ and $F_{SBS}(t)$ as the substrate input information at the culture time point t and solve the differential equation of Equation (4) using these pieces of substrate input information and the consumption rate $r_{SBS}$ of the substrate acquired from the AI model in the first step (S31), thereby calculating a substance amount $n_{SBS}$ in the substrate at the next culture time point (t+1), and, and can calculate a substrate concentration $C_{SBS}$ in the culture solution at the next culture time point (t+1) by substituting the substance amount into (Equation 5). The acquisition method for the bacterial cell mass B(t) at the culture time point t is as described above.

[Mathematical Equation 3]

$$\frac{dn_{SBS}}{dt} = -r_{SBS} \cdot B(t) + \frac{C_{Feed}}{MW_{SBS}} \cdot F_{SBS}(t) \qquad \cdots (\text{Equation 4})$$

$$C_{SBS} = \frac{n_{SBS} \cdot MW_{SBS}}{V} \qquad \cdots (\text{Equation 5})$$

However, the calculation equation for the substrate concentration used in the second step (S32) is not limited to such an example.

In the fourth step (S35), the CPU 11 acquires culture result information over a predetermined culture time obtained by executing the prediction cycle described above.

Here, the "predetermined culture time" is a culture time when the execution of the prediction cycle for a predetermined culture time determined in advance has been completed, and is a culture time until the completion when the prediction cycle has been completed by the end determination on the basis of the substrate concentration in the culture solution or the like.

The culture result information to be acquired may be, for example, a total production amount of the target object in the predetermined culture time, a total consumption amount of the substrate in the predetermined culture time, a balance (yield) of the target object with respect to the substrate in the predetermined culture time, or a plurality of them. In the present embodiment, the culture result information to be acquired is not limited.

As described above, the present method can be appropriately modified according to the mode of the AI model to be used. Specifically, the processing content of each step of the present method can be appropriately modified according to the content of the culture information which is the input information of the AI model and the content of the culture behavior speed index which is the output information of the AI model.

The culture information only need to include at least a concentration of the specific component and the specific culture condition in the culture vessel, and may include the temperature in the culture vessel, the hydrogen ion index (pH) of the culture solution in the culture vessel, and the oxygen transfer rate or the oxygen consumption rate as the specific culture condition, include the target object concentration, the byproduct concentration, and the substrate concentration as the concentration of the specific component, and include bacterial cell activity information and the byproduct state information.

In addition, the culture behavior speed index is a change rate of a specific component, and may be all or part of a generation rate of a target object, a generation rate of a byproduct, a consumption rate of a substrate, and a generation rate of carbon dioxide.

It is sufficient that the input information is updated for each culture time point according to the mode of the AI model, and various types of information for each culture time point are acquired so that the AI model can estimate the culture behavior speed index on the basis of the updated input information. For example, it is possible to use a substance balance equation for obtaining the substance amount of the byproduct at the next culture time point from the generation rate of the byproduct and the bacterial cell mass, or a substance balance equation for obtaining the substance amount of carbon dioxide at the next culture time point from the generation rate of carbon dioxide and the bacterial cell mass, and it is also possible to use calculation expressions for obtaining the concentration of the byproduct or the concentration of carbon dioxide at the next culture time point from these substance amounts.

Some or all of the above-described embodiments and modifications can also be identified as follows. However, the above-described embodiments and modifications are not limited to the following description.

<1> A culture behavior prediction method for predicting a culture behavior in a culture vessel in which a microorganism is cultured, in which

one or more processors capable of using a learned model that outputs a culture behavior speed index using culture information as an input executes:

a first step of acquiring a change rate of a specific component as the culture behavior speed index by inputting the culture information including a concentration of the specific component in the culture vessel and a specific culture condition at a certain culture time point to the learned model;
a second step of calculating the concentration of the specific component in the culture vessel at a next culture time point using at least the acquired change rate of the specific component;
a third step of acquiring the specific culture condition at the next culture time point; and
a fourth step of acquiring culture result information over a predetermined culture time by repeating a prediction cycle including the first step, the second step, and the third step related to each of the culture time points.

<2> The culture behavior prediction method according to <1>, in which

the specific component in the culture vessel is a target object generated in the culture behavior, and
the one or more processors,
in the first step, acquire a generation rate of the target object as the culture behavior speed index by inputting the culture information including the concentration of the target object to the learned model, and,
in the second step, calculate the concentration of the target object at the next culture time point at least using the acquired generation rate of the target object.

<3> The culture behavior prediction method according to <1> or <2>, in which

the specific culture condition includes a temperature in the culture vessel, a hydrogen ion index of a culture solution in the culture vessel, and an oxygen transfer rate or an oxygen consumption rate, and
the one or more processors,
in the first step, acquire a change rate of the specific component as the culture behavior speed index by inputting the culture information including the concentration of the specific component, the temperature, the hydrogen ion index, and the oxygen transfer rate or the oxygen consumption rate at the certain culture time point to the learned model, and
in the third step, acquire the specific culture condition including the temperature, the hydrogen ion index, and the oxygen transfer rate or the oxygen consumption rate at the next culture time point.

<4> The culture behavior prediction method according to <3>, in which

the one or more processors,
in the second step, calculate a specific bacterial cell mass of the microorganism at each culture time point on the basis of a culture elapsed time and the oxygen transfer rate or the oxygen consumption rate at each culture time point, and calculate the concentration of the specific component at the next culture time point using at least the

**EP 4 632 076 A1**

calculated bacterial cell mass and the acquired change rate of the specific component.

<5> The culture behavior prediction method according to any one of <1> to <4>, in which

the one or more processors,
in the first step, acquire, as the culture behavior speed index, a generation rate of the target object and a substrate consumption rate by inputting the culture information including the concentration of the target object generated by the culture behavior to the learned model,
in the second step, acquire substrate input information at the certain culture time point, and further calculate a substrate concentration in the culture vessel at the next culture time point using at least the substrate input information and the acquired substrate consumption rate, and
in the fourth step, determine continuation or end of repetition of the prediction cycle on a basis of the calculated substrate concentration.

<6> The culture behavior prediction method according to any one of <1> to <5>, in which

the culture information further includes bacterial cell activity information indicating a bacterial cell activity state of the microorganism by a categorical variable, and
the one or more processors,
in the first step, acquire the change rate of the specific component as the culture behavior speed index by inputting the culture information including the concentration of the specific component, the bacterial cell activity information, and the specific culture condition at the certain culture time point to the learned model, and,
in the second step, acquire the bacterial cell activity information at the next culture time point.

<7> The culture behavior prediction method according to <6>, in which

the specific culture condition includes an oxygen transfer rate or an oxygen consumption rate, and
the one or more processors
in the second step, calculate the bacterial cell activity information at the next culture time point on the basis of a culture elapsed time until the certain culture time point and the oxygen transfer rate or the oxygen consumption rate, and,
in the third step, acquire the specific culture condition including the oxygen transfer rate or the oxygen consumption rate at the next culture time point.

<8> The culture behavior prediction method according to any one of <1> to <7>, in which

the culture information further includes byproduct state information indicating a byproduct increase and decrease state in the culture vessel by a categorical variable, and
the one or more processors,
in the first step, acquire the generation rate of the byproduct as the change rate of the specific component by inputting the culture information including the concentration of the specific component, the byproduct state information, and the specific culture condition at the certain culture time point to the learned model, and,
in the second step, acquire the byproduct state information at the next culture time point using the acquired generation rate of the byproduct.

<9> The culture behavior prediction method according to any one of <1> to <8>, in which

the learned model outputs only the culture behavior speed index using only the culture information as an input, and
the culture information and the culture behavior speed index are configured by only an intensive variable or a dimensionless number, or both of them.

<10> A culture behavior prediction apparatus including at least a memory and the one or more processors,
the culture behavior prediction apparatus capable of executing the culture behavior prediction method according to any one of <1> to <9>.

Hereinafter, the above-described contents will be described in more detail with reference to examples. However, the description of the following examples does not add any limitation to the contents described above. Examples

In the present example, three types of AI models were constructed, and a prediction accuracy of the culture behavior prediction method according to the above-described embodiment was verified by using each of the AI models in the method. Here, the three types of AI models are referred to as an AI model M1, an AI model M2, and an AI model M3, the method using the AI model M1 is referred to as a first prediction method, the method using the AI model M2 is referred to as a second prediction method, and the method using the AI model M3 is referred to as a third prediction method.

Learning of each AI model was performed by a common learning algorithm using common data obtained in a culture experiment for producing an organic acid (target object) using aerobic bacteria as teacher data. A concrete method of acquiring the teacher data is as follows.

In the culture experiment, microorganisms (aerobic bacteria) were actually cultured using a predetermined culture tank (aeration stirring tank), and sampling was performed at intervals of several hours during the culture experiment. Then, various substance concentrations [g/L] in the culture solution were analyzed using high performance liquid chromatography (HPLC), and the wet bacterial cell concentration $B(t)/V(t)$ [g/L] was measured by measuring the wet bacterial cell weight per unit culture solution amount (volume). After the end of the experiment, the analysis and measurement results were formed into data at intervals of 5 minutes by numerical interpolation, and data at intervals of 5 minutes of the wet bacterial cell mass $B(t)$ [g] and the substance amount $n$ [mol] of various substances were derived from the culture solution amount $V(t)$ and the molar mass MW of the substance. Finally, the substance amount $n$ [mol], which was data at intervals of 5 minutes, was numerically differentiated at time $t$ [h] and divided by the wet bacterial cell mass $B(t)$ [g] to obtain the production rate or the consumption rate [mol/g/h] of the specific component (specific substance such as organic acid) per unit bacterial cell mass (wet bacterial cell weight).

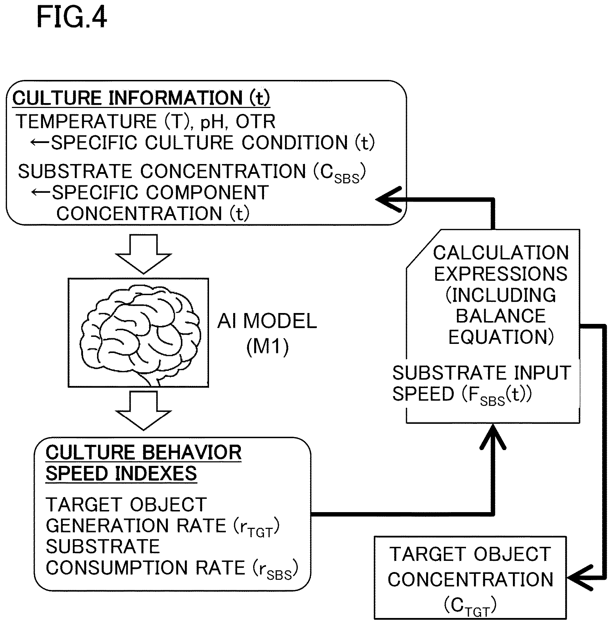

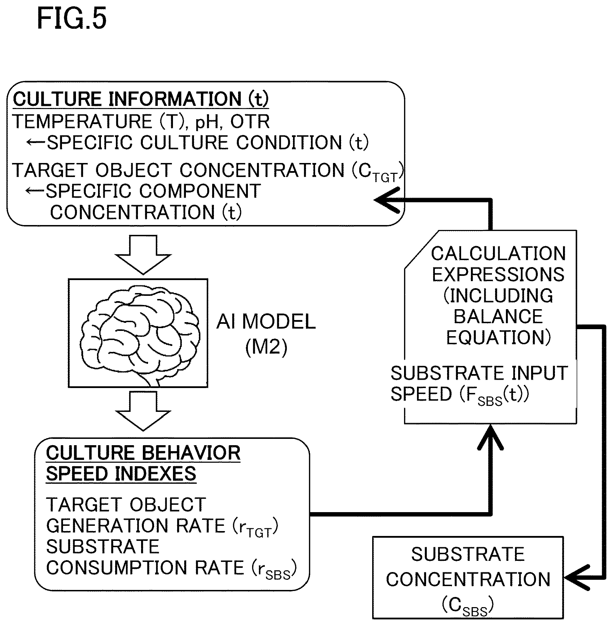

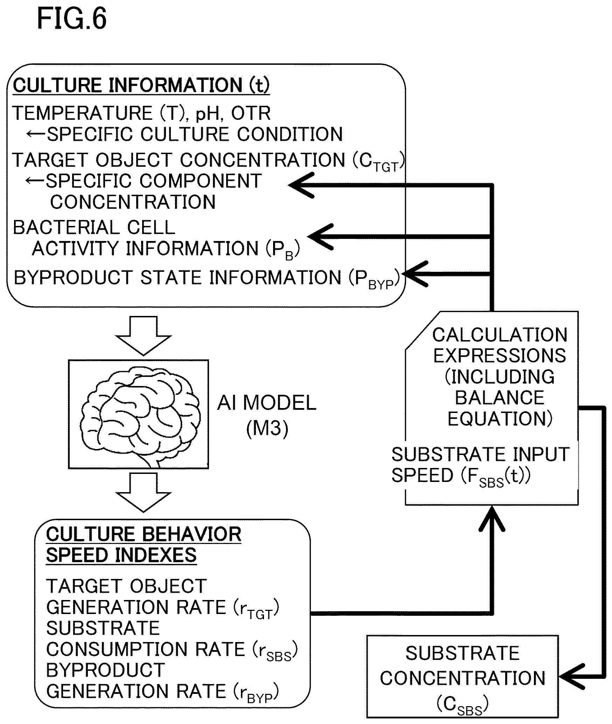

Fig. 4 is a diagram conceptually illustrating a flow of various types of information (data) in the first prediction method, Fig. 5 is a diagram conceptually illustrating a flow of various types of information (data) in the second prediction method, and Fig. 6 is a diagram conceptually illustrating a flow of various types of information (data) in the third prediction method.

As illustrated in Figs. 4, 5, and 6, each of the AI models is constructed such that input information (culture information) and output information (culture behavior speed index) are different from each other.

In the AI model M1, the temperature ($T$) of the culture solution, the hydrogen ion index (pH) of the culture solution and the oxygen transfer rate (OTR) as the specific culture condition, and the substrate concentration ($C_{SBS}$) in the culture solution as specific component concentration are defined as culture information, and the target object generation rate ($r_{TGT}$) and the substrate consumption rate ($r_{SBS}$) are defined as culture behavior speed indexes.

In the AI model M2, the culture behavior speed index is defined as the same as the AI model M1, and the culture information is defined as the same as the AI model M1 except that the target object concentration ($C_{TGT}$) is used instead of the substrate concentration ($C_{SBS}$).

The AI model M3 is different from the AI model M2 only in that bacterial cell activity information ($P_B$) and byproduct state information ($P_{BYP}$) are added to the culture information and that byproduct generation rate ($r_{BYP}$) is added to the culture behavior speed index, and the other contents of the culture information and the culture behavior speed index are identical to those in the AI model M2.

In the first prediction method illustrated in Fig. 4, in the pretreatment step (S30), the initial value of the substrate concentration ($C_{SBS}$), the initial bacterial cell concentration (BCI), the initial culture solution amount $V(0)$, and the substrate concentration ($C_{Feed}$) in the substrate input solution are acquired, the temperature ($T$), the pH and the OTR are acquired as the specific culture condition (0) at the culture time point (0), and the substrate input speed ($F_{SBS}(0)$) at the culture time point (0) is acquired.

In the first step (S31), the specific culture condition ($t$) and the substrate concentration ($C_{SBS}$) as the culture information at each culture time point ($t$) are input to the AI model, and the target object generation rate ($r_{TGT}$) and the substrate consumption rate ($r_{SBS}$) are acquired as the culture behavior speed indexes from the AI model.

As the culture information at the initial culture time point ($t=0$), the temperature ($T$), pH, and OTR, and the initial value of the substrate concentration ($C_{SBS}$) acquired in the pretreatment step (S30) are input to the AI model.

In the second step (S32), the substrate input speed ($F_{SBS}(t)$) is acquired as the substrate input information at the culture time point ($t$), the culture solution amount $V$ is calculated by solving the differential equation of the following substance balance equation (Equation 6) using the $F_{SBS}(t)$, and the specific bacterial cell mass $B(t)$ at the culture time point ($t$) is calculated by substituting the calculated culture solution amount $V$ and the OTR at the culture time point ($t$) into the following substance balance equation (Equation 7). Then, by solving the differential equation (Equation 1) described above using the calculated $B(t)$ and the target object generation rate ($r_{TGT}$) acquired in step (S31), the substance amount $n_{TGT}$ of the target object at the next culture time point ($t+1$) can be calculated, and the target object concentration ($C_{TGT}$) at the next culture time point ($t+1$) can be calculated using (Equation 2) described above.

In the following (Equation 7), the initial bacterial cell concentration (BCI=20) acquired in the pretreatment step (S30) is set, and k1, k2, and k3 in (Equation 3) described above are set to 0.18, 17.5, and 0.334.

[Mathematical Equation 4]

$$\frac{dV}{dt} = F_{SBS}(t) \quad \cdots \text{(Equation 6)}$$

$$\frac{B(t)}{V} = min\{20exp(0.18t), 17.5OTR^{0.334}\} \quad \cdots \text{(Equation 7)}$$

Furthermore, in the second step (S32), the substrate substance amount ($n_{SBS}$) at the next culture time point (t+1) is calculated by solving the differential equation of the described above Equation (4) using the $C_{Feed}$ and the $F_{SBS}(t)$ acquired as the substrate input information at the culture time point (t) and the substrate consumption rate ($r_{SBS}$) acquired from the AI model in the first step (S31), and the substrate concentration ($C_{SBS}$) in the culture solution at the next culture time point (t+1) is calculated by substituting the substance amount into (Equation 5) described above.

In the third step (S33), the temperature (T), the pH, and the OTR at the next culture time point (t+1) are acquired as specific culture condition (t+1).

A prediction cycle including the first step (S31), the second step (S32), and the third step (S33) at each culture time point (t) is repeatedly executed for a predetermined culture time determined in advance or until the substrate concentration ($C_{SBS}$) calculated in the second step (S32) is 0 or less.

The second prediction method illustrated in Fig. 5 is different from the first prediction method in that the initial value of the target object concentration ($C_{TGT}$) is acquired instead of the initial value of the substrate concentration ($C_{SBS}$) in the pretreatment step (S30), and the target object concentration ($C_{TGT}$) is input to the AI model instead of the substrate concentration ($C_{SBS}$) in the first step (S31), and the other processing contents of each of the steps are identical to those of the first prediction method.

The AI model M3 used in the third prediction method illustrated in Fig. 6 is different from the AI model M2 only in that bacterial cell activity information ($P_B$) and byproduct state information ($P_{BYP}$) are added to the culture information and that byproduct generation rate ($r_{BYP}$) is added to the culture behavior speed index, and the other contents of the culture information and the culture behavior speed index are identical to those in the AI model M2, as described above.

Therefore, the pretreatment step (S30) of the third prediction method is different from the pretreatment step (S30) of the first prediction method in that the initial values of the target object concentration ($C_{TGT}$), the bacterial cell activity information ($P_B$), and the byproduct state information ($P_{BYP}$) are respectively acquired instead of the initial value of the substrate concentration ($C_{SBS}$).

In the first step (S31), then the specific culture condition (t), the target object concentration ($C_{TGT}$), the bacterial cell activity information ($P_B$), and byproduct state information ($P_{BYP}$) as the culture information at each culture time point (t) are input to the AI model, and the target object generation rate ($r_{TGT}$), the substrate consumption rate ($r_{SBS}$), and the byproduct generation rate ($r_{BYP}$) are acquired as the culture behavior speed indexes from the AI model.

In addition, the second step (S32) of the third prediction method, the bacterial cell activity information ($P_B$) and the byproduct state information ($P_{BYP}$) at the next culture time point (t+1) are further acquired in addition to the processing contents of the same step of the first prediction method.

The bacterial cell activity information ($P_B$) at the next culture time point (t+1) is set to a value (0) indicating that the bacterial cell activity state is in the growth phase of bacterial cell when the left side is selected (smaller) in the min{} function of (Equation 7) described above, and is set to a value (1) indicating that the target object is in the production phase when the right side is selected (smaller).

The byproduct state information ($P_{BYP}$) at the next culture time point (t+1) is set to a value (0) indicating an increasing state of the byproduct when the byproduct generation rate ($r_{BYP}$) acquired from the AI model in the first step (S31) is 0 or more, and set to a value (1) indicating a decreasing state of the byproduct when the $r_{BYP}$ is smaller than 0.

The others are similar to those in the first prediction method.

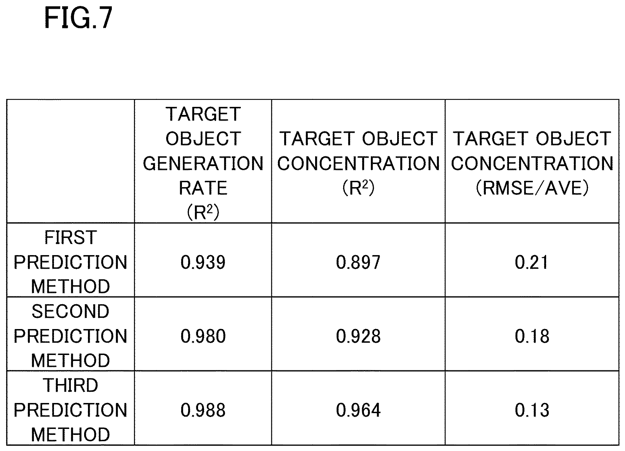

Fig. 7 is a table listing prediction accuracies of the first prediction method, the second prediction method, and the third prediction method.

In a column denoted by "target object generation rate ($R^2$)" in the table of Fig. 7, values of the prediction determination coefficient $R^2$ of the target object generation rate ($r_{TGT}$) per unit bacterial cell mass output from the AI models M1, M2, and M3 are respectively listed.

In addition, in the column denoted by "target object concentration ($R^2$)", values of the prediction determination

coefficient $R^2$ of the target object concentration ($C_{TGT}$) obtained as the culture result information from the target object concentration ($C_{TGT}$) calculated in the second step (S32) in the first prediction method, the second prediction method, and the third prediction method are respectively listed.

In the column denoted by "target object concentration (RMSE/AVE)", dimensionless values obtained by dividing the root-mean-square error RMSE of the target object concentration ($C_{TGT}$) at each culture time point calculated in the second step (S32) in the first prediction method, the second prediction method, and the third prediction method by the average value of the target object concentrations are respectively listed.

According to Fig. 7, the determination coefficient $R^2$ is 0.9 or more in all of the first prediction method (AI model M1), the second prediction method (AI model M2), and the third prediction method (AI model M3), and it is indicated that the target object generation rate can be estimated with high accuracy in any AI model.

Furthermore, since the determination coefficient $R^2$ is higher in the order of the first prediction method (AI model M1), the second prediction method (AI model M2), and the third prediction method (AI model M3), it is indicated that the prediction accuracy of the target object generation rate ($r_{TGT}$) is higher in using the target object concentration ($C_{TGT}$) as an input to the AI model than in using the substrate concentration ($C_{SBS}$), and the prediction accuracy of the target object generation rate ($r_{TGT}$) is higher in using the bacterial cell activity information (PB) and the byproduct state information (PBYP) in addition to the target object concentration ($C_{TGT}$).

In addition, with respect to the target object concentration ($C_{TGT}$) calculated from the target object generation rate ($r_{TGT}$) output from the AI model, it is indicated that the prediction accuracy is higher and the prediction error is smaller in the order of the first prediction method, the second prediction method, and the third prediction method. Therefore, it is indicated that the prediction accuracy of the target object concentration ($C_{TGT}$) is higher in using the target object concentration ($C_{TGT}$) as an input to the AI model than in using the substrate concentration ($C_{SBS}$), and the prediction accuracy of the target object concentration ($C_{TGT}$) is higher in using the bacterial cell activity information (PB) and the byproduct state information (PBYP) in addition to the target object concentration ($C_{TGT}$).

As described above, according to the present example, it is demonstrated that the prediction accuracy of the culture behavior prediction method according to the above-described embodiment is high regardless of which of the three types of AI models is used.

This application claims priority based on Japanese Patent Application No. 2022-194670 filed on December 6, 2022, which is entirely incorporated herein by reference.

REFERENCE SIGNS LIST

10   Information processing apparatus (culture behavior prediction apparatus)
11   CPU
12   Memory
13   Input and output I/F
14   Communication unit
15   Display device
16   Input device

**Claims**

1. A culture behavior prediction method for predicting a culture behavior in a culture vessel in which a microorganism is cultured, wherein
   one or more processors capable of using a learned model that outputs a culture behavior speed index using culture information as an input executes:

   a first step of acquiring a change rate of a specific component as the culture behavior speed index by inputting the culture information including a concentration of the specific component in the culture vessel and a specific culture condition at a certain culture time point to the learned model;
   a second step of calculating the concentration of the specific component in the culture vessel at a next culture time point using at least the acquired change rate of the specific component;
   a third step of acquiring the specific culture condition at the next culture time point; and
   a fourth step of acquiring culture result information over a predetermined culture time by repeating a prediction cycle including the first step, the second step, and the third step related to each of the culture time points.

2. The culture behavior prediction method according to claim 1, wherein

the specific component in the culture vessel is a target object generated in the culture behavior, and the one or more processors,

in the first step, acquire a generation rate of the target object as the culture behavior speed index by inputting the culture information including the concentration of the target object to the learned model, and,

in the second step, calculate the concentration of the target object at the next culture time point at least using the acquired generation rate of the target object.

3.  The culture behavior prediction method according to claim 1 or 2, wherein

the specific culture condition includes a temperature in the culture vessel, a hydrogen ion index of a culture solution in the culture vessel, and an oxygen transfer rate or an oxygen consumption rate, and the one or more processors,

in the first step, acquire a change rate of the specific component as the culture behavior speed index by inputting the culture information including the concentration of the specific component, the temperature, the hydrogen ion index, and the oxygen transfer rate or the oxygen consumption rate at the certain culture time point to the learned model, and,

in the third step, acquire the specific culture condition including the temperature, the hydrogen ion index, and the oxygen transfer rate or the oxygen consumption rate at the next culture time point.

4.  The culture behavior prediction method according to claim 3, wherein

the one or more processors,

in the second step, calculate a specific bacterial cell mass of the microorganism at each culture time point on the basis of a culture elapsed time and the oxygen transfer rate or the oxygen consumption rate at each culture time point, and calculate the concentration of the specific component at the next culture time point using at least the calculated bacterial cell mass and the acquired change rate of the specific component.

5.  The culture behavior prediction method according to any one of claims 1 to 4, wherein

the one or more processors,

in the first step, acquire, as the culture behavior speed index, a generation rate of the target object and a substrate consumption rate by inputting the culture information including the concentration of the target object generated by the culture behavior to the learned model,

in the second step, acquire substrate input information at the certain culture time point, and further calculate a substrate concentration in the culture vessel at the next culture time point using at least the substrate input information and the acquired substrate consumption rate, and

in the fourth step, determine continuation or end of repetition of the prediction cycle on a basis of the calculated substrate concentration.

6.  The culture behavior prediction method according to any one of claims 1 to 5, wherein

the culture information further includes bacterial cell activity information indicating a bacterial cell activity state of the microorganism by a categorical variable, and

the one or more processors,

in the first step, acquire, the change rate of the specific component as the culture behavior speed index by inputting the culture information including the concentration of the specific component, the bacterial cell activity information, and the specific culture condition at the certain culture time point to the learned model, and,

in the second step, acquire the bacterial cell activity information at the next culture time point.

7.  The culture behavior prediction method according to claim 6, wherein

the specific culture condition includes an oxygen transfer rate or an oxygen consumption rate, and the one or more processors,

in the second step, calculate the bacterial cell activity information at the next culture time point on a basis of a culture elapsed time until the certain culture time point and the oxygen transfer rate or the oxygen consumption rate, and

in the third step, acquire the specific culture condition including the oxygen transfer rate or the oxygen consumption rate at the next culture time point.

8. The culture behavior prediction method according to any one of claims 1 to 7, wherein

the culture information further includes byproduct state information indicating a byproduct increase and decrease state in the culture vessel by a categorical variable, and
the one or more processors,
in the first step, acquire the generation rate of the byproduct as the change rate of the specific component by inputting the culture information including the concentration of the specific component, the byproduct state information, and the specific culture condition at the certain culture time point to the learned model, and,
in the second step, acquire the byproduct state information at the next culture time point using the acquired generation rate of the byproduct.

9. The culture behavior prediction method according to any one of claims 1 to 8, wherein

the learned model outputs only the culture behavior speed index using only the culture information as an input, and
the culture information and the culture behavior speed index are configured by only an intensive variable or a dimensionless number, or both of them.

10. A culture behavior prediction apparatus comprising at least a memory and the one or more processors, the culture behavior prediction apparatus capable of executing the culture behavior prediction method according to any one of claims 1 to 9.

# FIG.1

MICROORGANISM
SPECIFIC
BACTERIAL CELL

TARGET
PRODUCT

BYPRODUCT

CULTURE MEDIUM
COMPONENT
(SUBSTRATE)

SUBSTRATE
INPUT
SOLUTION

CULTURE
SOLUTION

TEMPERATURE

pH

AIR (OXYGEN)

# FIG.2

INFORMATION PROCESSING APPARATUS
(CULTURE BEHAVIOR
PREDICTION APPARATUS)

10

11
CPU

13
INPUT AND OUTPUT
I／F

15
DISPLAY
DEVICE

12
MEMORY

14
COMMUNICATION
UNIT

16
INPUT
DEVICE

FIG.3

```
                    ┌─────────────┐
                    │    START    │
                    └──────┬──────┘
                           │
            ┌──────────────▼──────────────┐
            │    PRETREATMENT STEP        │  ~ S30
            └──────────────┬──────────────┘
                           │
        ┌──────────────────▼──────────────┐
        │                                 │
   ┌────┼──────────────────┐              │
   │    │      FIRST STEP   │  ~ S31      │
   │    └──────────────────┘              │
   │                │                     │
   │    ┌───────────▼──────┐              │
   │    │    SECOND STEP   │  ~ S32       │
   │    └──────────────────┘              │
   │                │                     │
   │    ┌───────────▼──────┐              │
   │    │    THIRD STEP    │  ~ S33       │
   │    └──────────────────┘              │
   │                │                     │
   │         ┌──────▼──────┐  ~ S34       │
   │    ┌────┤ DOES PREDICTION ├───       │
   │    │    │ CYCLE END?      │          │
   │    └────┤                 ├───       │
   │   NO    └──────┬──────────┘          │
   └─────────────   │ YES                 │
                    │                     │
            ┌───────▼──────┐              │
            │  FOURTH STEP │  ~ S35       │
            └───────┬──────┘              │
                    │                     │
             ┌──────▼──────┐              │
             │     END     │              │
             └─────────────┘              │
```

# FIG.4

**CULTURE INFORMATION (t)**
TEMPERATURE (T), pH, OTR
    ←SPECIFIC CULTURE CONDITION (t)
SUBSTRATE CONCENTRATION ($C_{SBS}$)
    ←SPECIFIC COMPONENT
        CONCENTRATION (t)

AI MODEL
(M1)

**CULTURE BEHAVIOR**
**SPEED INDEXES**

TARGET OBJECT
GENERATION RATE ($r_{TGT}$)
SUBSTRATE
CONSUMPTION RATE ($r_{SBS}$)

CALCULATION
EXPRESSIONS
(INCLUDING
BALANCE
EQUATION)

SUBSTRATE INPUT
SPEED ($F_{SBS}(t)$)

TARGET OBJECT
CONCENTRATION
($C_{TGT}$)

# FIG.5

**CULTURE INFORMATION (t)**
TEMPERATURE (T), pH, OTR
  ←SPECIFIC CULTURE CONDITION (t)
TARGET OBJECT CONCENTRATION ($C_{TGT}$)
  ←SPECIFIC COMPONENT
    CONCENTRATION (t)

AI MODEL
(M2)

**CULTURE BEHAVIOR
SPEED INDEXES**

TARGET OBJECT
GENERATION RATE ($r_{TGT}$)
SUBSTRATE
CONSUMPTION RATE ($r_{SBS}$)

CALCULATION
EXPRESSIONS
(INCLUDING
BALANCE
EQUATION)

SUBSTRATE INPUT
SPEED ($F_{SBS}(t)$)

SUBSTRATE
CONCENTRATION
($C_{SBS}$)

# FIG.6

**CULTURE INFORMATION (t)**

TEMPERATURE (T), pH, OTR
  ←SPECIFIC CULTURE CONDITION
TARGET OBJECT CONCENTRATION ($C_{TGT}$)
  ←SPECIFIC COMPONENT
    CONCENTRATION
BACTERIAL CELL
  ACTIVITY INFORMATION ($P_B$)
BYPRODUCT STATE INFORMATION ($P_{BYP}$)

AI MODEL
(M3)

**CULTURE BEHAVIOR
SPEED INDEXES**

TARGET OBJECT
GENERATION RATE ($r_{TGT}$)
SUBSTRATE
CONSUMPTION RATE ($r_{SBS}$)
BYPRODUCT
GENERATION RATE ($r_{BYP}$)

CALCULATION
EXPRESSIONS
(INCLUDING
BALANCE
EQUATION)

SUBSTRATE INPUT
SPEED ($F_{SBS}(t)$)

SUBSTRATE
CONCENTRATION
($C_{SBS}$)

# FIG.7

| | TARGET OBJECT GENERATION RATE ($R^2$) | TARGET OBJECT CONCENTRATION ($R^2$) | TARGET OBJECT CONCENTRATION (RMSE/AVE) |
|---|---|---|---|
| FIRST PREDICTION METHOD | 0.939 | 0.897 | 0.21 |
| SECOND PREDICTION METHOD | 0.980 | 0.928 | 0.18 |
| THIRD PREDICTION METHOD | 0.988 | 0.964 | 0.13 |

## INTERNATIONAL SEARCH REPORT

| International application No. |
| --- |
| **PCT/JP2023/043573** |

**A.    CLASSIFICATION OF SUBJECT MATTER**

*C12Q 1/02*(2006.01)i; *C12M 1/00*(2006.01)i; *C12M 1/34*(2006.01)i
FI:    C12Q1/02; C12M1/00 C; C12M1/34 B

According to International Patent Classification (IPC) or to both national classification and IPC

**B.    FIELDS SEARCHED**

Minimum documentation searched (classification system followed by classification symbols)

C12Q1/02; C12M1/00; C12M1/34

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2003-235544 A (HITACHI, LTD.) 26 August 2003 (2003-08-26)<br>claims, examples, paragraphs [0017]-[0025] | 1-10 |
| X | JP 2022-146429 A (KANEKA CORP.) 05 October 2022 (2022-10-05)<br>claims, examples, paragraph [0026] | 1-10 |

☐ Further documents are listed in the continuation of Box C.      ☑ See patent family annex.

| | |
| --- | --- |
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "D"    document cited by the applicant in the international application | "X"    document of particular relevance; the claimed invention cannot be considered novel or cannot be considered to involve an inventive step when the document is taken alone |
| "E"    earlier application or patent but published on or after the international filing date | |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
| --- | --- |
| **13 February 2024** | **27 February 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2023/043573**

| Patent document cited in search report | | | Publication date (day/month/year) | Patent family member(s) | Publication date (day/month/year) |
|---|---|---|---|---|---|
| JP | 2003-235544 | A | 26 August 2003 | (Family: none) | |
| JP | 2022-146429 | A | 05 October 2022 | (Family: none) | |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022194670 A